# EUROPEAN PATENT APPLICATION

(11) **EP 1 610 187 A2**
(43) Date of publication of application: **28.12.2005**
(21) Application number: 05253154.8
(22) Date of filing: 21.05.2005
(51) Int. Cl.: G03G 15/00

(54) **High voltage supply device for developer**

(30) Priority: 25.05.2004 KR 2004037534
(71) Applicant: SAMSUNG ELECTRONICS CO., LTD., Suwon-si, Gyeonggi-do (KR)
(72) Inventor: Kim, Min-seon, Suwon-si, Gyeonggi-do (KR)
(74) Representative: Robinson, Ian Michael

(57) **Abstract**

A high voltage supply device for a developer unit including a first sub high voltage generating unit (210) having at least one Zener diode (ZD1, ZD2) for generating a first sub high voltage from a main high voltage formed by superposing first DC and AC voltages, and for applying the first sub high voltage to a developing agent supply roller (120). The high voltage supply device also includes a second sub high voltage generating unit (230) having at least one Zener diode (ZD3, ZD4, ZD5) for generating a second sub high voltage from the main high voltage and applying the second sub high voltage to a developing roller (180). The high voltage supply device further includes a first current stabilizing unit (250) connected between the first sub high voltage generating unit (210) and a ground, and a second current stabilizing unit (270) connected between the second sub high voltage generating unit (230) and the ground.

## Description

The present invention relates to an electrophotograph type color image forming device. More particularly, the present invention relates to a device for stably supplying a high voltage formed by superposing DC and AC voltages to a plurality of developer units for respective colors, regardless of load change, for color printing or copying.

Non-contact type color image forming devices comprise fixed type developer units which maintain a predetermined development gap of, for example, 0.2 mm without contacting a developing roller with a photosensitive drum or without separating the developing roller from the photosensitive drum. The fixed type developer units can solve various problems occurring in contact type developer units that use an eccentric cam, a cam drive motor, and an electronic clutch to transfer the developer for accommodating respective developing agents of yellow, magenta, cyan, and black, at the time of developing. In the aforementioned non-contact type color image forming devices, a voltage supply device for a developer unit comprises a high voltage supply source for supplying high voltages to a developing roller and a developing agent supply roller, a fixed contact terminal of a fixed type developer unit, and a printing circuit board (PCB) for transferring a high voltage output from the high voltage supply source to the fixed contact terminal of the developer unit.

In the non-contact type color image forming devices using the fixed type developer unit, the developer is not in contact with or separated from the photosensitive drum, so that deterioration of image quality and shortened lifetime of the photosensitive drum due to contact shock can be suppressed. In addition, the voltage supply device for the developer unit uses the PCB, so that a high voltage transfer interval resulting in complicated wire harnesses can be minimized.

However, in the above devices, a structure of the wire harness for supplying a voltage from the high voltage source, a structure of connecting lines patterned in the PCB, and a structure of high voltage contacts for switching a high voltage are still complicated. Therefore, when the developers are exchanged, there is a problem in that high voltage noise occurs and switching reliability is lowered at the high voltage contacts.

Accordingly, a need exists for a system and method for stably supplying a high voltage to a plurality of developer units for respective colors, regardless of load changes and having minimal high voltage noise.

According to the present invention there is provided an apparatus and method as set forth in the appended claims. Preferred features of the invention will be apparent from the dependent claims, and the description which follows.

The present invention provides a high voltage supply source for color printing or copying in which at least two sub high voltages having different voltage levels are generated from a main high voltage using a Zener diode and are then supplied to a developer unit.

The present invention further provides a high voltage supply source for stably supplying a high voltage formed by superposing DC and AC voltages to a plurality of developer units, regardless of load changes.

According to an aspect of the present invention, a high voltage supply device for a developer unit is provided comprising a first sub high voltage generating unit having at least one Zener diode for generating a first sub high voltage from a main high voltage formed by superposing a first DC and AC voltage, and applying the first sub high voltage to a developing agent supply roller, and a second sub high voltage generating unit having at least one Zener diode for generating a second sub high voltage from the main high voltage and applying the second sub high voltage to a developing roller. The voltage supply device further comprises a first current stabilizing unit connected between a first sub high voltage input contact of the first sub high voltage generating unit and the developing agent supply roller and a ground, and a second current stabilizing unit connected between a second sub high voltage input contact of the second sub high voltage generating unit and the developing roller and the ground.

According to another aspect of the present invention, a high voltage supply device is provided for a developer unit comprising a main high voltage unit for generating a main high voltage formed by superposing first DC and AC voltages, a switch unit for switching in accordance with a predetermined developing timing to supply the main high voltage to one of the developer units for respective colors, and a plurality of sub high voltage units for generating stable first and second sub high voltages from the main high voltage supplied from the switch unit, regardless of load changes, and for supplying the stable first and second sub high voltages to a developing agent supply roller and a developing roller, respectively.

The above and other features and advantages of the present invention will become more apparent by describing in detail exemplary embodiments thereof with reference to the attached drawings in which:
Figure 1 is a block diagram illustrating a high voltage supply device of a developer unit according to an embodiment of the present invention;
Figure 2 is a circuit diagram illustrating a detailed construction of a sub high voltage unit of Figure 1 according to an embodiment of the present invention;
Figures 3A to 3C are wave form diagrams illustrating a main high voltage and first and second sub high voltages of Figure 2 according to an embodiment of the present invention; and
Figure 4 is a circuit diagram for illustrating a method of calculating a resistance value for stabilizing a Zener current of Figure 2.

Throughout the drawings, like reference numerals will be understood to refer to like parts, components and structures.

The present invention will now be described in greater detail with reference to the accompanying drawings.

A high voltage supply device for a developer unit according to an embodiment of the present invention is preferably provided with a non-contact type color image forming device. The non-contact type color image forming device comprises a fixed developer unit to maintain a predetermined developing gap of, for example, 0.2 mm without contacting a developing roller with a photosensitive drum and without separating the developing roller from the photosensitive drum. In order to perform a developing process in a color laser beam printer using the fixed developer unit, of which, the electric field is inversely proportional to the developing gap, a toner moves from the developing roller to the photosensitive drum. The toner moves from the developing roller to the photosensitive drum by supplying a high voltage formed by superposing AC and DC voltages, to the developing agent supply roller and the developing roller. When an AC voltage is applied, a moving direction of the toner is determined by a developing vector, that is, a DC component of a developing bias - electric potential of a photosensitive drum surface. When using a negatively charged toner, if the developing vector is negatively charged, the toner of the developing roller moves to a photosensitive drum. If the developing vector is positively charged, the toner of the developing roller does not move.

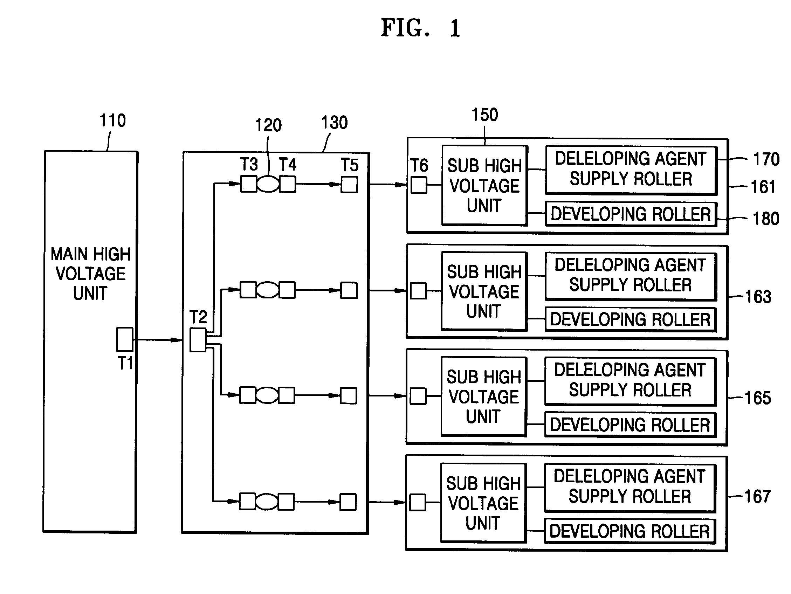

Figure 1 is a block diagram illustrating a high voltage supply device of a developer unit according to an embodiment of the present invention. The high voltage supply device for the developer unit comprises a main high voltage unit 110, a switch unit 130, and a sub high voltage unit 150. It is preferable that the sub high voltage unit 150 be placed within the developer units 161, 163, 165, and 167 for respective colors so as to reduce the number of high voltage output contacts in the switch unit 130 and the number of high voltage input contacts in the developer units 161, 163, 165, and 167.

The main high voltage unit 110 generates a main high voltage by superposing first DC and AC voltages. A proper range of the first DC and AC voltages constituting the main high voltage is set to generate first and second sub high voltages required for a developing roller 180, and a developing agent supply roller 170, respectively, from the main high voltage. The main high voltage generated from the main high voltage unit 110 is supplied to the switch unit 130 through a wiring harness between high voltage output and input contacts T1 and T2, respectively.

The switch unit 130, having a solenoid 120 that includes the high voltage input and output contacts T3 and T4, respectively, selectively switches the main high voltage supplied from the high voltage input contact T2 in accordance with a predetermined developing timing to supply a sub high voltage generated from the main high voltage unit 110 to the developer units 161, 163, 165, and 167 for the respective colors. The sub high voltage is provided to the developer units 161, 163, 165, and 167 through a wiring harness between high voltage output and input contacts T5 and T6, respectively. The solenoid can be comprised of, but not limited to, devices such as a relay.

The sub high voltage unit 150 generates the stable first and second sub high voltages, regardless of load changes, from the main high voltage supplied from the switch unit 130 to the high voltage input contact point T6, and supplies the generated first and second sub high voltages to the developing agent supply roller 170 and the developing roller 180, respectively.

According to the aforementioned construction, by generating and providing at least two sub high voltages from one main high voltage supplied from the main high voltage unit 110 to the developer units 161, 163, 165, and 167 for the respective colors, it is possible to drastically reduce the number of high voltage wire harnesses, pattern connection lines, and high voltage switch contacts required for supplying a voltage from the main high voltage unit 110 to the developer units 161, 163, 165, and 167 for the respective colors.

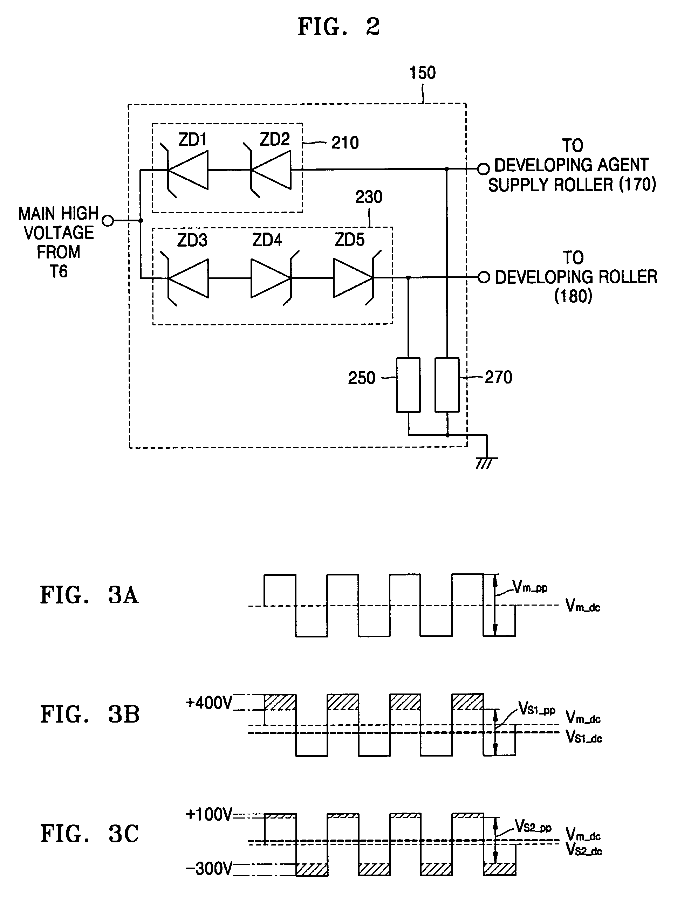

Figure 2 is a circuit diagram illustrating a detailed construction example of the sub high voltage unit 150 of Figure 1 according to an embodiment of the present invention. The sub high voltage unit 150 comprises a first sub high voltage generating unit 210, a second sub high voltage generating unit 230, a first current stabilizing unit 250, and a second current stabilizing unit 270. Operation of the sub high voltage unit 150 will now be described in greater detail with reference to the waveform diagrams shown in Figures 3A to 3C.

Referring to Figure 2, a main high voltage that is formed by superposing first DC and AC voltages V_{m_dc} and V_{m_pp}, respectively, as shown in Figure 3A, is applied to the first and second sub high voltage generating units 210 and 230.

The first sub high voltage generating unit 210 comprises at least one Zener diode, or more than one Zener diode connected in series. In an embodiment of the present invention, the first sub high voltage generating unit 210 comprises the first and second backward Zener diodes ZD1 and ZD2. The first and second Zener diodes ZD1 and ZD2 have a Zener voltage of 200V, for example. The first and second Zener diodes ZD1 and ZD2 therefore, provide a function of clipping 400V in a positive direction for the first AC voltage V_{m_pp}. As a result, the first sub high voltage generating unit 210 generates the first sub high voltage shown in FIG. 3B by superposing the first sub DC and AC voltages V_{s1_dc} and V_{s1_pp}, respectively, and supplies the first sub high voltage to the developing agent supply roller 170. The first sub DC voltage V_{s1_dc} is decreased by 200V compared to the first DC voltage V_{m_dc}. The first sub AC voltage V_{s1_pp} is decreased by 400 Vₚₚ compared to the first AC voltage V_{m_pp}.

The second sub high voltage generating unit 230 comprises at least one Zener diode, or more than one Zener diode connected in series. In an embodiment of the present invention, the second sub high voltage generating unit 230 comprises the third backward Zener diode ZD3, and fourth and fifth forward Zener diodes ZD4 and ZD5. The third Zener diode ZD3 has a Zener voltage of 100V, for example. The fourth and fifth Zener diodes ZD4 and ZD5 have Zener voltages of 100V and 200V, for example, respectively. The third Zener diode ZD3 therefore, provides a function of clipping 100V in a positive direction for the first AC voltage V_{m_pp}. The fourth and fifth Zener diodes ZD4 and ZD5 provide a function of clipping 300V in a negative direction for the first AC voltage V_{m_pp}. As a result, the second sub high voltage generating unit 230 generates the second sub high voltage shown in Figure 3C by superposing the second sub DC and AC voltages V_{s2_dc} and V_{s2_pp}, respectively, and supplies the second sub high voltage to the developing roller 180. The second sub DC voltage V_{s2_dc} is increased by 100V compared to the first DC voltage V_{m_dc}. The second sub AC voltage V_{s2_pp} is decreased by 400 Vₚₚ compared to the first AC voltage V_{m_pp}.

The second sub high voltage supplied to the developing roller 180 is higher by 100V in the case of the second sub DC voltage, and is decreased by 400V in the case of the second sub AC voltage, as is the first sub high voltage supplied to the developing agent supply roller 170. In an embodiment of the present invention, the difference between the first and second sub high voltages is 300V in a DC voltage, and the first and the second sub high voltages in an AC voltage are equal to each other.

The number and the connecting direction of the Zener diodes ZD1, ZD2, ZD3, ZD4, and ZD5 comprising the first and second sub high voltage generating units 210 and 230 are not limited to the above embodiments. Various modifications in the number and the connecting directions of the Zener diodes are possible according to a size of the first DC and AC voltages constituting the main high voltage, and a size of the first and second sub high voltages required for the developing agent supply roller 170 and the developing roller 180.

The first current stabilizing unit 250 comprises at least one resistor (not shown) connected in parallel to a load resistor or resistance (not shown) that includes a developing agent supply roller 170 resistance, a toner resistance, or a developing roller 180 resistance. The second current stabilizing unit 270 comprises at least one resistor (not shown) connected in parallel to a load resistor or resistance (not shown) that includes a developing agent supply roller 170 resistance, a toner resistance, or a developing roller 180 resistance. The first and second current stabilizing units 250 and 270 have a function of allowing the Zener diodes ZD1, ZD2, ZD3, ZD4, and ZD5 to conduct a predetermined range of Zener current to facilitate the regulation of the Zener diode, even in a case wherein the load resistance varies as the developing agent supply roller 170 resistance, the developing roller 180 resistance, or the ground terminal changes. As a result, it is possible to prevent the deterioration of print quality due to damage of electrostatic latent images on the photosensitive drum, or due to a discharge from the developing roller 180 to the photosensitive drum caused by an unregulated voltage exceeding a rating voltage applied to the developing agent supply roller 170 and the developing roller 180.

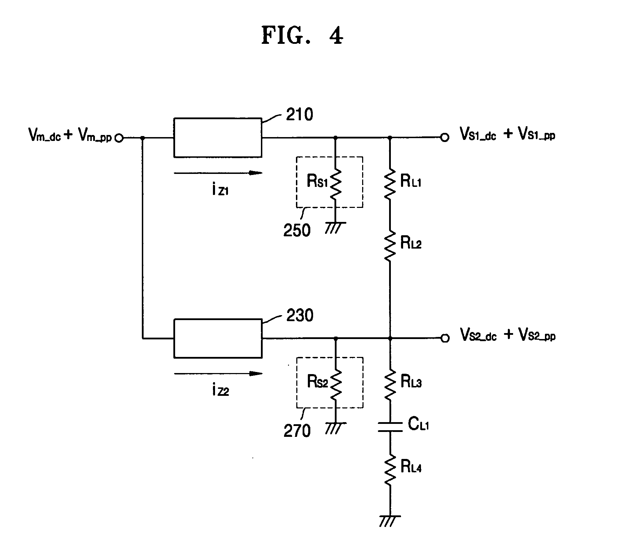

Figure 4 is a diagram illustrating a method of calculating resistance values of resistors comprising the first and second current stabilizing units 250 and 270 for stabilizing a Zener current of Figure 2.

A Zener current i_{z1} is conducted by the Zener diodes ZD1 and ZD2 comprising the first sub high voltage generating unit 210, and a Zener current i_{z2} is conducted by the Zener diodes ZD3, ZD4, and ZD5 comprising the second sub high voltage generating unit 230. Further, resistance R_{L1}, R_{L2}, and R_{L3} represent a developing agent supply roller 170 resistance, a toner resistance, and a developing roller 180 resistance, respectively. A capacitance C_{L1} is formed in a predetermined gap between the developing roller 180 and the photosensitive drum. The resistance R_{L4} represents a resistance of the photosensitive drum. Accordingly, a combined impedance of R_{L1} to R_{L4} and C_{L1} can be used to represent a load resistance R_{L} which can vary in accordance with changes of toner characteristics, aging phenomenon, or changes of the ground terminal.

A value of the first resistor Rₛ₁ comprising the first current stabilizing unit 250 and which can be connected in parallel to the load resistance R_{L}, is determined by a minimum Zener current i_{z1_min} of the Zener diodes ZD1 and ZD2 comprising the first sub high voltage generating unit 210, and the first AC voltage V_{m_pp} value of the main high voltage. The minimum Zener current i_{z1_min}, which is a lowest current value required for the normal regulation of the Zener diodes, can be obtained from a part specification of the Zener diodes. For example, when the minimum Zener current i_{z1_min} is 1 mA and the first AC voltage V_{m_pp} is 2 KV, a value of the first resistor Rₛ₁ is 2M ohm. Therefore, even if the load resistance R_{L} varies, a value of the total load resistor Rₜ, that is, a parallel resistance value of the first resistor Rₛ₁ and the load resistance R_{L}, is smaller than a value of the first resistor Rₛ₁, that is, 2M ohm. Therefore, a current greater than the minimum Zener current i_{z1_min} of the Zener diode can be conducted by the Zener diodes ZD1 and ZD2.

A value of the second resistor Rₛ₂ comprising the second current stabilizing unit 270 can be determined by the minimum Zener current i_{z2_min} of the Zener diodes ZD3, ZD4, and ZD5 comprising the second sub high voltage generating unit 230 and the first AC voltage V_{m_pp} value of the main high voltage.

Although the aforementioned exemplary embodiment illustrates only the first sub high voltage for the developing agent supply roller 170 and the second sub high voltage for the developing roller 180, a third sub high voltage for a developing agent layer restriction blade (not shown) can also be generated by using the same method and apparatus. In addition, the present invention may be adapted to a laser beam printer using a multi pass method or a single pass method.

As described above, according to embodiments of the present invention, it is possible to generate the first and second sub high voltages from the main high voltage using a plurality of Zener diodes in the developer unit to thereby reduce high voltage noise at the time of exchanging the developer by additionally connecting a resistance for stabilizing the Zener current between the output and ground terminals of the first and second sub high voltages. Embodiments of the present invention further enhance the reliability in the switching operation of high voltage contacts, and enhance the ability to normally regulate the Zener diodes, even if load resistance varies by changes in the developing agent supply roller 170 resistance, the developing roller 180 resistance, or a ground line resistance. As a result, since the stable first and second sub high voltages are supplied to the developing agent supply roller 170 and the developing roller 180, the developing operation can be stably performed, so that it is possible to improve print quality and to extend a lifetime of the photosensitive drum.

Although a few preferred embodiments have been shown and described, it will be appreciated by those skilled in the art that various changes and modifications might be made without departing from the scope of the invention, as defined in the appended claims.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A high voltage supply device for a developer unit, comprising:
a first sub high voltage generating unit (210) having at least one Zener diode (ZD1, ZD2) for generating a first sub high voltage from a main high voltage, wherein the main high voltage is formed by superposing first DC and AC voltages, and applying the first sub high voltage to a developing agent supply roller (120);
a second sub high voltage generating unit (230) having at least one Zener diode (ZD3, ZD4, ZD5) for generating a second sub high voltage from the main high voltage and applying the second sub high voltage to a developing roller (180);
a first current stabilizing unit (250) connected between a ground and a coupling between the first sub high voltage generating unit (210) and the developing agent supply roller (120); and
a second current stabilizing unit (270) connected between the ground and a coupling between the second sub high voltage generating unit (230) and the developing roller (180).

2. The high voltage supply device for a developer unit according to claim 1, wherein each of the first and second current stabilizing units comprises at least one resistor (R_{S1}, R_{S2}).

3. The high voltage supply device for a developer unit according to claim 2, wherein a value of the resistor (R_{S1}, R_{S2}) is determined by a minimum value of a Zener diode current rating and the first AC voltage value.

4. The high voltage supply device for a developer unit according to any preceding claim, wherein the first and second sub high voltage generating units (210, 230) and the first and second current stabilizing units (250, 270) comprise a bias board.

5. The high voltage supply device for a developer unit according to claim 4, wherein the bias board is disposed in the developer unit.

6. A high voltage supply device for a developer unit, comprising:
a main high voltage unit (110) for generating a main high voltage formed by superposing first DC and AC voltages;
a switch unit (130) for switching in accordance with a predetermined developing timing to supply the main high voltage to one of a plurality of developer units; and
a plurality of sub high voltage units (150) for generating stable first and second sub high voltages from the main high voltage supplied from the switch unit (130) regardless of a load change and for supplying the stable first and second sub high voltages to a developing agent supply roller (120) and a developing roller (180), respectively.

7. The high voltage supply device for a developer unit according to claim 6, wherein each of the sub high voltage units (150) comprises:
a first sub high voltage generating unit (210) having at least one Zener diode (ZD1, ZD2) for generating the first sub high voltage from the main high voltage and applying the first sub high voltage to the developing agent supply roller (120);
a second sub high voltage generating unit (230) having at least one Zener diode (ZD3, ZD4, ZD5) for generating the second sub high voltage from the main high voltage and applying the second sub high voltage to the developing roller (180);
a first current stabilizing unit (250) connected between a ground and a coupling between the first sub high voltage generating unit (210) and the developing agent supply roller (120); and
a second current stabilizing unit (270) connected between the ground and a coupling between the second sub high voltage generating unit (230) and the developing roller (180).

8. The high voltage supply device for a developer unit according to claim 7, wherein each of the first and second current stabilizing units comprises at least one resistor (R_{S1}, R_{S2}).

9. The high voltage supply device for a developer unit according to claim 8, wherein a value of the resistor (R_{S1}, R_{S2}) is determined by a minimum value of a Zener diode current rating and the first AC voltage value.

10. The high voltage supply device for a developer unit according to any of claims 6 to 7, wherein the sub high voltage units comprise a bias board.

11. The high voltage supply device for a developer unit according to claim 10, wherein the bias board is disposed in the developer unit.

12. A method of providing a high voltage for a developer unit, comprising the steps:
generating a first sub high voltage from a main high voltage, wherein the main high voltage is formed by superposing first DC and AC voltages, and applying the first sub high voltage to a developing agent supply roller (120);
generating a second sub high voltage from the main high voltage and applying the second sub high voltage to a developing roller (180);
stabilizing the first sub high voltage applied to the developing agent supply roller (120) during an exchange of developer; and
stablizing the second sub high voltage applied to the developing roller during an exchange of developer, wherein the first sub high voltage and the second sub high voltage are generated at a developer unit.
